# EUROPEAN PATENT APPLICATION

(11) **EP 1 240 865 A2**
(43) Date of publication of application: **18.09.2002**
(21) Application number: 02394032.3
(22) Date of filing: 14.03.2002
(51) Int. Cl.: A61B 5/00

(54) **Method for prevention/rehabilitation customization**

(30) Priority: 14.03.2001 US 808742
(71) Applicant: PeakCare LLC, San Diego, California 92130 (US)
(72) Inventor: Leeds, Gary, California 92067 (US); Leeds, Jordan, San Diego, California 92130 (US)
(74) Representative: Shortt, Peter Bernard

(57) **Abstract**

A system and method for creating a personal occupational program at a website includes prevention and rehabilitation information that is specifically directed to an individual's work environment and occupational specialty in order to foster worksite wellness. In operation, an end user will input personal data as background for his/her personal occupational program. A medical expert may, or may not, help customize the program that includes prevention and rehabilitation sections by prescribing treatment exercises for the individual end user. In general, sections of the program provide information on education, activities of daily living, exercise and specific safe work activities. Once the program is prepared, the end user may retrieve information in his/her program over the internet, in a printout, on a CD or on a videotape.

## Description

### FIELD OF THE INVENTION

The present invention pertains generally to methods and systems for presenting information about worksite wellness to an individual end user at his/her worksite. More particularly, the present invention pertains to presentations that are accessible by an individual end user over a global computer network, such as the internet. The present invention is particularly, but not exclusively, useful as a method and system for creating a personal occupational program that includes prevention and rehabilitation information that is specifically directed to an individual end user's work environment and occupational specialty.

### BACKGROUND OF THE INVENTION

Worksite wellness is generally defined as the prevention of physical and mental illness and injury and the promotion of overall health and safety within the workplace. It is an issue of vital importance to any business concern. As a practical matter, it is equally important to both the employer and the employee. Within the ambit of worksite wellness are such concerns as: morale of the workforce, productivity, quality of products and services, and the overall health of individuals in the workplace. The various aspects of these concerns are far-reaching and include, in part, considerations of mental health, behavioral and psychological issues, as well as physical and medical ailments. Suffice it to say, the subject of worksite wellness is extensive. In the final analysis, however, the bottom line issue concerns the overall well being of the individual worker.
The key to effectively addressing the issues associated with worksite wellness is actually twofold. First, if possible, it is preferable to anticipate a potentially debilitating situation and take the appropriate corrective action before there are any adverse consequences. Hopefully, the appropriate corrective action can be taken by the individual worker without the involvement of others. Second, if necessary, it is desirable that the individual worker's situation be evaluated and properly referred to appropriate professional help as early as possible. In both instances, education of the individual worker is essential.

In order to properly educate a workforce about worksite wellness, there are several considerations which need to be taken into account. Specifically, these considerations involve the individual needs of the particular worker, the specific requirements of the occupational tasks that are performed by the particular worker, and the physical and mental requirements imposed on the worker and the environmental conditions of the worksite. Further, there are considerations which affect other workers, such as support and administrative personnel, as well as the owner/employer.

In light of the above it is an object of the present invention to provide a method and system for establishing an occupational program to foster worksite wellness which can be customized and individually tailored to the needs of a particular end user. Another object of the present invention is to provide a method and system for establishing a personalized occupational program to foster worksite wellness which provides both prevention and rehabilitation information that is tailored to the needs of the particular end user. Still another object of the present invention is to provide a method and system for establishing a personalized occupational program to foster worksite wellness which is instantaneously responsive to the inquiries of an individual worker. Still another object of the present invention is to provide a method and system for establishing a personalized occupational program to foster worksite wellness which can be delivered to the particular end user over the internet, in a handout, a videotape or a CD. Yet another object of the present invention is to provide a method and system for establishing a customized occupational program to foster worksite wellness which is easy to use, simple to access or install and comparatively cost effective.

### SUMMARY OF THE PREFERRED EMBODIMENTS

In accordance with the present invention, a system for creating a personalized occupational program, that is tailored to foster worksite wellness, essentially requires effective communications between a provider (a source for information), an intermediary (owner/employer), and an individual end user (the worker/employee). More specifically, as intended for the present invention, communications between the provider and the intermediary are conducted over a global computer network, such as the internet. Internal company communications between the intermediary and the end user, however, are to be conducted over a local communications system, such as wiring between a central console and personal computers in the company. Within this framework, communications between the end user and the provider will be conducted over the internet through the intermediary.
As intended for the present invention, communications between the provider and the end user is preferably a worksite wellness website that is linked to, or incorporated a part of, the intermediary's home website. This worksite wellness website is operated and maintained by the intermediary in cooperation with the provider. Additionally, the worksite wellness website will pertain directly to the end user, and to his/her occupational well being. The worksite wellness website for the present invention is a vehicle for an end user to create his/her personal occupational program.

In order to do this, the end user will first login to the website. If the end user is a first time user, the end user will input personal data (My Profile). With the personal data, a personal occupational program (My Programs) is created for the end user. Further, the system and method of the present invention allows the end user to validate his/her personal occupational program for the specific needs of the end user, and to modify his/her personal occupational program as necessary. If the end user is a return user or a verified user, the end user will bypass the input of personal data (My Profile) and go directly to his/her personal occupational program (My Programs).

The personal occupational program will include a prevention section and a rehabilitation section. Both the prevention section and the rehabilitation section each include information in four categories. In more detail, these four categories are Education, Activities of Daily Living (ADL), Exercise, and Specific Safe Work Activities. Within each category, there is information pertaining to selected subject areas, such as safety, work hazards, environmental illnesses, worksite injuries, and performance motivation.

In another aspect of the present invention, a particular end user's personal occupational program may be further customized by a medical expert identified by the provider or by the intermediary. Specifically, the medical expert may include additional medical information specific to the needs and condition of the end user. As contemplated for the present invention, the medical expert may also prescribe treatment or rehabilitation exercises for the end user to perform due to his/her condition.

As intended for the present invention, the personal occupational program can be delivered to the end user in several ways. Specifically, the information provided in the personal occupational program can be delivered to the end user preferably over the internet, in streaming video form or in storyboard form. Alternatively, the personal occupational program may also be delivered as a printout, on a CD or on videotape.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Fig. 1 is a schematic representation of the system of the present invention showing the interrelationship of the parties through their respective communications links with each other;
Fig. 2 is a functional activity flowchart of the website operation of the present invention for use by an end user to create a personal occupational program; and
Fig. 3 is a diagram of the interrelationships between categories within a personal occupational program.

### DESCRIPTION OF THE PREFERRED EMBODIMENT

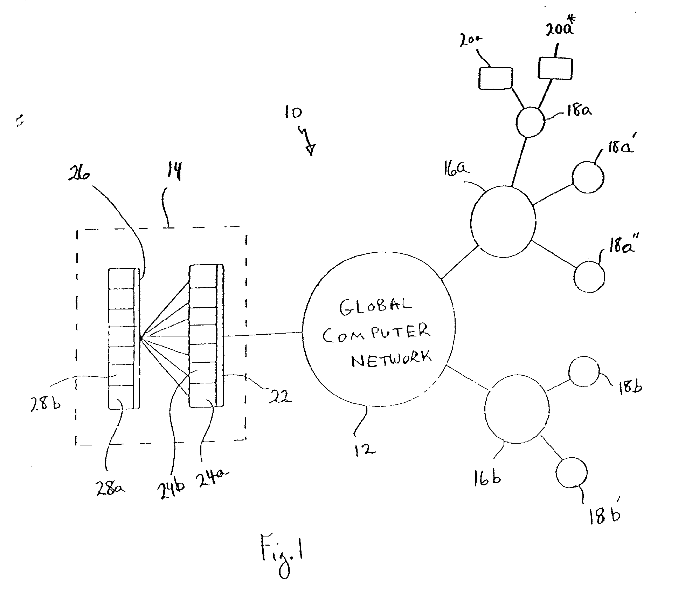

Referring to Fig. 1, a system for establishing a customized audio/visual presentation for on-site use, that is designed to foster worksite wellness in accordance with the present invention, is shown and generally designated 10. As shown, the system 10 involves a communications scheme with links over a global computer network, such as the internet 12. Specifically, these communications links for the system 10 are established between a provider 14, an intermediary 16 and a worksite 18. With reference to Fig. 1, it is to be appreciated that the intermediaries 16a and 16b are only exemplary, and that many such intermediaries 16 can be included. Specifically, an intermediary 16 will be any owner/employer, organization or group of affiliates that incorporates the system 10 into his/her/their business for the purpose of providing employees with effective information about worksite wellness. For the system 10, the worksites 18a, 18a' and 18a" that are associated with the intermediary 16a, as well as the worksites 18b and 18b' that are associated with the intermediary 16b are only exemplary. As envisioned for the system 10 of the present invention, there may be as many worksites 18 associated with an intermediary 16 as are desired by the particular intermediary 16. Further, each worksite 18 should be as accessible to the intended end user as is possible. Preferably, each worksite 18 will incorporate a personal computer 20 for use by the end user. The particular personal computers 20a and 20a* shown associated with the worksite 18a are only exemplary.
The format for communications over the system 10 will, perhaps, be best appreciated with reference to the provider 14. As shown, it is possible to access an information module 22 through the provider 14. Actually, the provider 14 is able to offer several different types of information modules 22. The particular type of information module 22 offered by the provider 14 will depend on the nature of the industry involved and, more specifically, on the occupational descriptor 24 for which the information module 22 was prepared. Typical occupational descriptors 24 will include classifications chosen from a group that can include a supervisory descriptor, a clerical descriptor, a strenuous manual labor descriptor, a non-strenuous manual labor descriptor, a custodial descriptor, and any other appropriate descriptor. Thus, for example, one information module 22 may be specifically prepared with an occupational descriptor 24a for supervisory personnel, while another information module 22 may be specifically prepared with an occupational descriptor 24b for persons engaged in strenuous manual labor activities.

As also shown in Fig. 1, according to its occupational descriptor 24, each information module 22 can be directed toward an occupational program 26. Further, each occupational program 26 will include digital excerpts for various subject areas 28 such as safety, work hazards, environmental illnesses, worksite injuries, and performance motivation. For example, one occupational program 26 may pertain to a subject area 28a that addresses safety issues while another occupational program 26 will pertain to a subject area 28b that involves performance motivation issues. Again, by way of example, the consequence of the above is that a warehouse employee (end user), whose job involves lifting and moving heavy objects, can use a personal computer 20 at his/her worksite 18, and through the intermediary 16 gain access over the internet 12 to the provider 14. This particular end user could then identify an occupational descriptor 24 (e.g. occupational descriptor 24b) for an information module 22, and then choose an occupational program 26 with safety as its subject area 28 (e.g. subject area 28a). As will be appreciated by the skilled artisan, within the scheme, there are many commutations and permutations to the information modules 22 and occupational programs 26 that are available to the end user over the system 10.

It is an important aspect of the present invention that the intermediary 16 be able to select the particular information modules 22 that are to be made available to end users at worksites 18 operated by the intermediary 16. With this in mind, it is thus possible for the intermediary 16 to tailor the information modules 22 and occupational programs 26 for inclusion in a worksite wellness stratagem that will benefit his/her employees. Of equal importance is the ability of the end user (employee) to customize his/her information module 22 and occupational program 26. Specifically, as envisioned for the system 10 of the present invention, an end user at a personal computer 20, at a worksite 18, is able to provide personal information that will specifically customize the particular occupational program 26 for his/her needs. The resultant audio/visual presentation will then be in conformance with, and in accordance with, the personal data, the occupational program 26, and the selected information module 22. Further, at the worksite 18, the end user will be able to validate the audio/visual presentation and modify it as necessary.

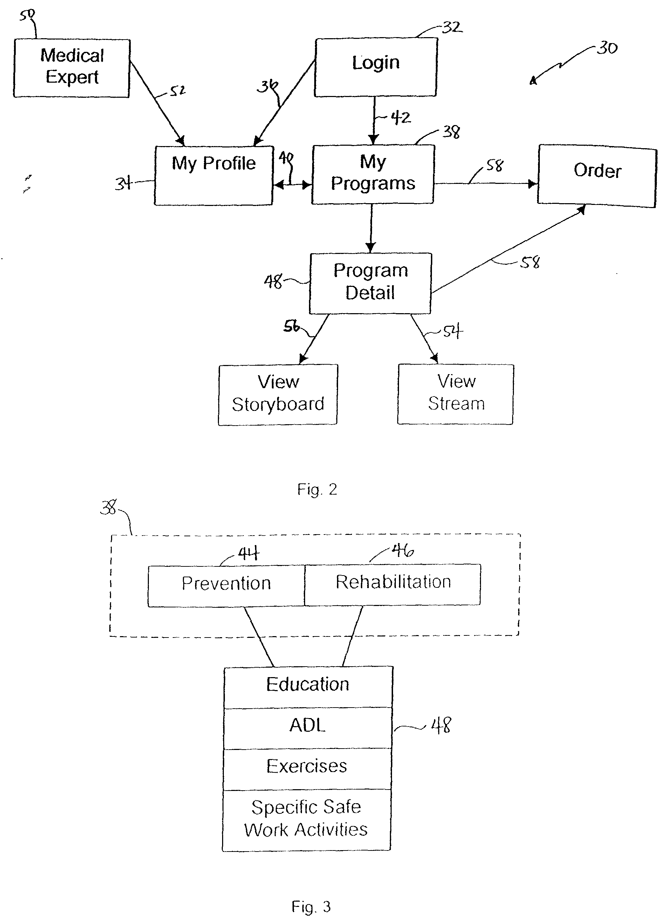

In an alternative embodiment for the present invention, system 10 includes a worksite wellness website, that is generally designated 30 in Fig. 2, which is designed to allow an end user to create a personal occupational program tailored to his/her needs and occupational specialty. The website 30 is operated and maintained by the intermediary 16 (employer) in cooperation with the provider 14. It is important to note that the end user can access the website 30, and thus his/her personal occupational program, at a worksite 18, at home or at any other place having an internet connection.

Fig. 2 is a functional activity flowchart of the website 30 of system 10. As indicated in Fig. 2, to create a personal occupational program, the end user will first Login 32 at the home page of the worksite wellness website 30. If the end user is a first time user, the end user will input personal data into the My Profile 34 of the website 30 as indicated by arrow 36. Personal data, for example, may include age, weight, height, gender, occupation, industry, activities, and illnesses of the end user. The scope of the present invention also includes personal data regarding health-related activities and conditions such as stress management, nutrition, smoker's sensation and diabetes. This list is not exclusive, but only by way of example. In any case, after the end user has completed My Profile 34 with personal data, the end user can validate My Profile 34 and then advance to My Programs 38 (personal occupational program) of the website 30 as indicated by arrow 40. If the end user is a return user or a verified user, the end user will directly go from the Login 32 to My Programs 38 of the website 30 as indicated by arrow 42.

In accordance with the present invention, My Programs 38 has a prevention section 44 and a rehabilitation section 46, as shown in Fig. 3. Both the prevention and the rehabilitation sections 44 and 46 each include a Program Detail 48. Program Detail 48 has four categories as shown in Fig. 3. Specifically, these categories are Education, Activities of Daily Living (ADL), Exercises and Specific Safe Work Activities. Thus, the end user can find information relevant to each subject category. For example, in Education, the end user can find information on injury prevention and recovery. Additionally, descriptions of common injuries related to the occupational specialty of the end user can also be found. The end user may then view the information in every category or he/she may view only the categories that are of interest to the end user.

As intended for this alternative embodiment of the present invention, as shown in Fig. 2, a medical expert 50 that is identified by a provider 14 or by an intermediary 16 (shown in Fig. 1) can review an end user's My Profile 34 as indicated by arrow 52. This medical expert 50 may be a physical therapist, a medical doctor, an occupational therapist or any other medical specialist the provider 14 or intermediary 16 deems appropriate for an individual end user. After reviewing the profile of the end user, the medical expert 50 may find it necessary, for the well being of the end user, to prescribe a set of treatment or rehabilitation exercises developed specifically for the end user by the medical expert 50. The medical expert 50 will incorporate these treatment exercises into the end user's My Programs 38. Alternatively, the medical expert 50 will change the number of sets of the predetermined exercises listed in the Exercise category of the My Programs 38 of the end user. In addition to the treatment exercises, the medical expert 50 may also include in My Programs 38 additional medical information pertaining to the end user's specific condition.

It is important to note that the end user may view his/her My Programs 38 as many times as desired. Of equal importance, the end user may modify his/her My Programs 38 by changing the personal data in My Profile 34. To do this, the end user can easily return to My Profile 34 from My Programs 38 as indicated by arrow 40. My Programs 38 can be modified as often as necessary to reflect the occupational changes of the end user. As implied above, the medical expert 50 may also modify My Programs 38 at any time.

As contemplated for this worksite wellness website 30 of the present invention, the end user may view or obtain his/her personal occupational program by various deliverables. Specifically, it is preferred that these deliverables for My Programs 38 are on the website 30 by streaming video as indicated by arrow 54 or by storyboard as indicated by arrow 56. Alternatively, the end user has the option to print out his/her occupational program or order a CD or a videotape that will have his/her personal occupational program as indicated by arrow 58.

While the particular Method for Prevention/Rehabilitation Customization as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as described in the appended claims.

## Claims

1. A method for fostering worksite wellness which comprises the steps of:
establishing a worksite wellness website, said worksite wellness website being operated and maintained by an intermediary in cooperation with a provider;
selectively providing personal data by an end user to said worksite wellness website;
reviewing said personal data of said end user;
selectively prescribing a plurality of treatment exercises developed by a medical expert for use at said worksite wellness website, wherein said plurality of treatment exercises are responsive to said reviewing step;
incorporating said personal data and said plurality of treatment exercises to establish a personal occupational program for use by said end user; and
retrieving said personal occupational program from said worksite wellness website through said intermediary by said end user.

2. A method as recited in claim 1 further comprising the step of validating said personal occupational program for adaptation by said end user.

3. A method as recited in claim 2 further comprising the step of modifying said personal occupational program in response to said validating step and said reviewing step.

4. A method as recited in claim 3 wherein said modifying step is accomplished by said end user.

5. A method as recited in claim 3 wherein said modifying step is accomplished by said medical expert.

6. A method as recited in claim 1 wherein said personal occupational program includes a prevention section and a rehabilitation section.

7. A method as recited in claim 6 wherein said prevention section has information pertaining to selected subject areas, and wherein said subject areas are selected from a group including education, activities of daily living, exercise and specific safe work activities.

8. A method as recited in claim 6 wherein said rehabilitation section includes said plurality of treatment exercises prescribed by said medical expert for use by said end user, and further includes information pertaining to selected subject areas, and wherein said subject areas are selected from a group including education, activities of daily living, exercise and safe work activities.

9. A method as recited in claim 1 wherein said retrieving step is accomplished by deliverables, and wherein said deliverables are selected from a group including streaming over the internet, printing a handout, ordering a videotape, and ordering a CD.

10. A system to foster worksite wellness by establishing a personal occupational program for use by an end user which comprises:
a provider for creating a worksite wellness program having a plurality of information modules, with each said information module pertaining to a specific occupational specialty and for combining a plurality of occupational programs within each said information module;
a means for connecting said provider in communication with an intermediary via an internet connection;
a means for allowing said intermediary to present said plurality of information modules for accessibility for said end user;
a means for customizing a specific said information module into a personal occupational program with personal data by said end user;
a medical source for evaluating said personal data of said end user, and for selectively tailoring said personal occupational program to include a plurality of treatment exercises developed by said medical source; and
a means for retrieving said personal occupational program from said provider through said intermediary, said personal occupational program being presented in conformance with and in accordance with said personal data and said plurality of treatment exercises.

11. A system as recited in claim 10 wherein said personal occupational program includes a prevention section and a rehabilitation section.

12. A system as recited in claim 11 wherein said prevention section and said rehabilitation section each include information pertaining to selected subject areas, and wherein said subject areas are chosen from a group including education, activities of daily living, exercises and specific safe work activities.

13. A system as recited in claim 11 wherein said rehabilitation section includes said plurality of treatment exercises prescribed by said medical source for said particular end user to perform.

14. A system as recited in claim 10 wherein said means for retrieving said personal occupational program is selected from a group consisting of internet streaming, printout, CD, and videotapes.

15. A method for using a worksite wellness program operated and maintained by an intermediary in cooperation with a provider which comprises the steps of:
customizing said worksite wellness program into a personal occupational program with personal data by an end user;
retrieving said personal occupational program by said provider and through said intermediary;
reviewing said personal data of said end user by a medical expert;
selectively prescribing a plurality of treatment exercises in said personal occupational program to said end user developed by said medical expert; and
selectively modifying said personal occupational program by said end user and said medical expert.

16. A method as recited in claim 15 wherein said personal occupational program includes a prevention section and a rehabilitation section.

17. A method as recited in claim 16 wherein said prevention section and said rehabilitation section each includes information pertaining to selected subject areas, and wherein said subject areas are selected from a group including education, activities of daily living, exercise and specific safe work activities.

18. A method as recited in claim 16 wherein said rehabilitation section includes said plurality of treatment exercises prescribed by said medical expert.

19. A method as recited in claim 15 wherein said retrieving step is accomplished by deliverables, and wherein said deliverables are selected from a group including streaming over the internet, printing a handout, ordering a videotape and ordering a CD.

20. A network system adapted to provide a customised personal occupation program for one or more remote users, the sysem comprising:
a server interfaceable with one or more of a service provider and an intermediary, the server having a plurality of information modules, each module pertaining to a specific occupational speciality and adapted for combining with one or more of a plurality of occupational programs within each of said information modules,
communication means adapted to enable a user to access one or more of said information modules on the server over a network connection so as to provide information specific to the user to the server, the user being associatable with the intermediary,
means for customizing an occupational program to the personal requirements of the user, the customization being determined on the basis of information provided by the user and an analysis of that provided information by a third party, and
means for providing the customized occupational program to the user in an audiovisual format.
